# EUROPÄISCHE PATENTSCHRIFT

(11) **EP 4 197 998 B1**
(45) Veröffentlichungstag und Bekanntmachung des Hinweises auf die Patenterteilung: **22.05.2024**
(21) Anmeldenummer: 21215348.0
(22) Anmeldetag: 17.12.2021
(51) Int. Cl.: C07F 15/00, C07C 45/50

(54) **PT-SIXANTPHOS-IOD-KOMPLEX UND PT-SIXANTPHOS-BROM-KOMPLEX**
PT-SIXANTPHOS-IODINE COMPLEX AND PT-SIXANTPHOS-BROMIUM COMPLEX
COMPLEXE PT-SIXANTPHOS-IODE ET COMPLEXE PT-SIXANTPHOS-BROME

(43) Veröffentlichungstag der Anmeldung: 21.06.2023
(73) Patentinhaber: Evonik Oxeno GmbH & Co. KG, 45772 Marl (DE)
(72) Erfinder: Schneider, Carolin, 40789 Monheim am Rhein (DE); Jackstell, Ralf, 18106 Rostock (DE); Beller, Matthias, 18211 Ostseebad Nienhagen (DE); Franke, Robert, 45772 Marl (DE)
(74) Vertreter: Evonik Patent Association

(56) Entgegenhaltungen:
- VAN DUREN RUBEN ET AL: "Platinum-catalyzed hydroformylation of terminal and internal octenes", DALTON TRANSACTIONS, Nr. 10, 1. Januar 2007 (2007-01-01), Seite 1053, XP055922345, Cambridge ISSN: 1477-9226, DOI: 10.1039/b615428j

## Beschreibung

Die vorliegende Erfindung betrifft einen Pt-Sixantphos-lod-Komplex und Pt-Sixantphos-Brom-Komplex, sowie deren Verwendung zur Katalyse einer Hydroformylierungsreaktion.

In P. Meessen et al., Journal of Organometallic Chemistry, 551, (1998), 165-170 wird der Einsatz von SixantphosPtCl₂ zur Hydroformylierung von Methyl-3-pentenoat beschrieben.

In R. van Duren et al., Dalton Transactions, 2007, 1053-1059, "Platinum-catalyzed hydroformylation of terminal and internal octenes", werden Pt/Sn-Katalysatoren beschrieben.

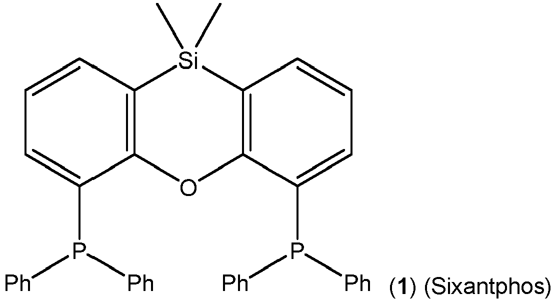

Der vorliegende Erfindung lag die Aufgabe zugrunde, einen neuen Komplex bereitzustellen. Der Komplex soll hierbei bei der Katalyse von Hydroformylierungsreaktionen gegenüber dem im Stand der Technik beschriebenen Komplex eine gesteigerte Ausbeute liefern.

Diese Aufgabe wird gelöst durch einen Komplex gemäß Anspruch 1.

Komplex umfassen:
a) Pt;
b) einen Liganden entsprechend der Formel (I): 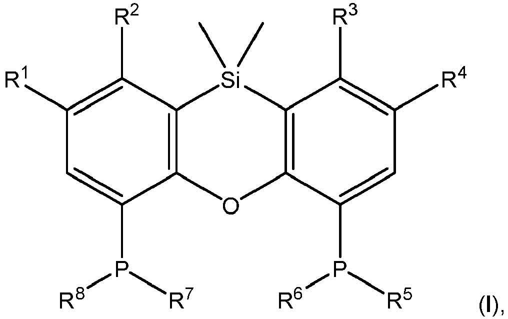 wobei R¹, R², R³, R⁴, R⁵, R⁶, R⁷, R⁸ ausgewählt sind aus: -H, -(C₁-C₁₂)-Alkyl, -(C₆-C₂₀)-Aryl;
c) einen lod-Liganden oder Brom-Liganden.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform stehen R⁵, R⁶, R⁷, R⁸ für -(C₆-C₂₀)-Aryl.

In einer Ausführungsform stehen R⁵, R⁶, R⁷, R⁸ für -Ph.

In einer Ausführungsform stehen R¹ und R⁴ für -H.

In einer Ausführungsform stehen R² und R³ für -H.

In einer Ausführungsform stehen weist die Verbindung gemäß der Formel (I) die Struktur (1) auf:

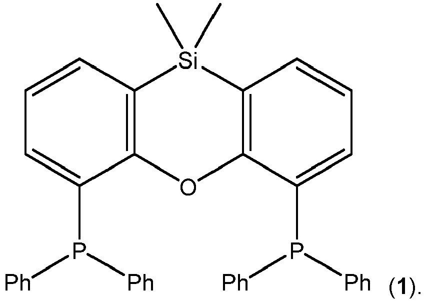

In einer Ausführungsform weist der Komplex genau einen Liganden entsprechend der Formel (I) auf.

In einer Ausführungsform weist der Komplex mindestens zwei lod-Liganden auf.

In einer Ausführungsform weist der Komplex genau zwei lod-Liganden auf.

In einer Ausführungsform weist der Komplex mindestens zwei Brom-Liganden auf.

In einer Ausführungsform weist der Komplex genau zwei Brom-Liganden auf.

Neben dem Komplex an sich, wird auch dessen Verwendung zur Katalyse einer Hydroformylierungsreaktion beansprucht.

Verwendung eines zuvor beschriebenen Komplexes zur Katalyse einer Hydroformylierungsreaktion.

Im Folgenden soll die Erfindung anhand von Ausführungsbeispielen näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und 1-Octen in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 18 h bei 80 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Variation des Halogens

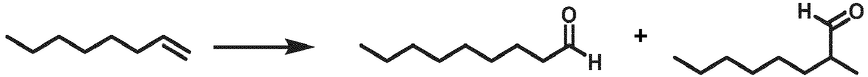

### Reaktionsbedingungen:

1.0 mmol 1-Octen, 0.5 mol% PtX₂, 2,0 Äquivalente Ligand (1), Lösungsmittel: Toluol, p(CO/H₂): 40 bar, T: 80 °C, t: 18 h.

### Ausbeuten:

| Ligand | Halogen | Ausbeute [%] |
|---|---|---|
| 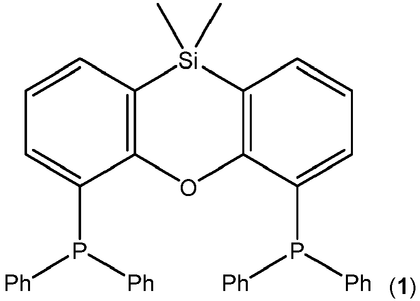 | I / Br / Cl | 98 / 97 / <1 |

Wie die Versuchsergebnisse zeigen, wird die Aufgabe durch den erfindungsgemäßen Komplex gelöst.

## Patentansprüche

1. Komplex umfassen:
a) Pt;
b) einen Liganden entsprechend der Formel (I): 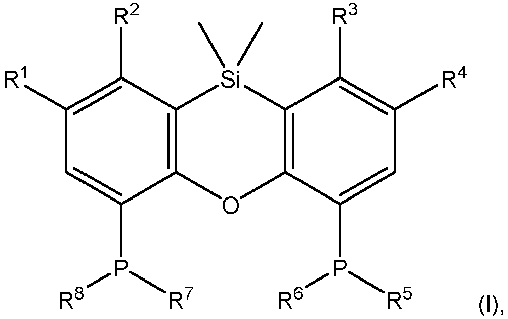 wobei R¹, R², R³, R⁴, R⁵, R⁶, R⁷, R⁸ ausgewählt sind aus: -H, -(C₁-C₁₂)-Alkyl, -(C₆-C₂₀)-Aryl;
c) einen lod-Liganden oder Brom-Liganden.

2. Komplex nach Anspruch 1,
wobei R⁵, R⁶, R⁷, R⁸ für -(C₆-C₂₀)-Aryl stehen.

3. Komplex nach einem der Ansprüche 1 oder 2,
wobei R⁵, R⁶, R⁷, R⁸ für -Ph stehen.

4. Komplex nach einem der Ansprüche 1 bis 3,
wobei R¹ und R⁴ für -H stehen.

5. Komplex nach einem der Ansprüche 1 bis 4,
wobei R² und R³ für -H stehen.

6. Komplex nach einem der Ansprüche 1 bis 5,
wobei die Verbindung gemäß der Formel **(I)** die Struktur **(1)** aufweist:

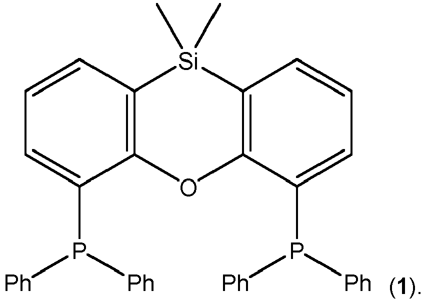

7. Komplex nach einem der Ansprüche 1 bis 6,
wobei der Komplex genau einen Liganden entsprechend der Formel (I) aufweist.

8. Komplex nach einem der Ansprüche 1 bis 7,
wobei der Komplex mindestens zwei lod-Liganden aufweist.

9. Komplex nach Anspruch 8,
wobei der Komplex genau zwei lod-Liganden aufweist.

10. Komplex nach einem der Ansprüche 1 bis 7,
wobei der Komplex mindestens zwei Brom-Liganden aufweist.

11. Komplex nach Anspruch 10,
wobei der Komplex genau zwei Brom-Liganden aufweist.

12. Verwendung eines Komplexes nach einem der Ansprüche 1 bis 11 zur Katalyse einer Hydroformylierungsreaktion.

## Claims

1. Complex comprising:
a) Pt;
b) a ligand corresponding to formula (I): 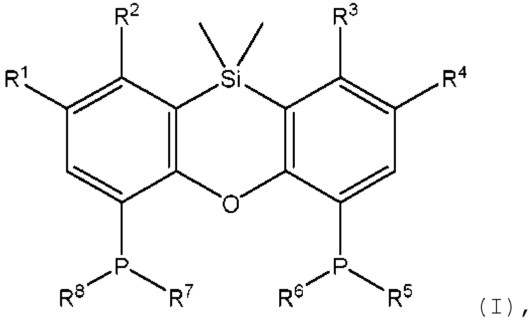 where R¹, R², R³, R⁴, R⁵, R⁶, R⁷, R⁸ are selected from: -H, - (C₁-C₁₂)-alkyl, -(C₆-C₂₀)-aryl;
c) an iodine ligand or bromine ligand.

2. Complex according to Claim 1,
wherein R⁵, R⁶, R⁷, R⁸ are -(C₆-C₂₀)-aryl.

3. Complex according to either of Claims 1 and 2, wherein R⁵, R⁶, R⁷, R⁸ are -Ph.

4. Complex according to any of Claims 1 to 3,
wherein R¹ and R⁴ are -H.

5. Complex according to any of Claims 1 to 4,
wherein R² and R³ are -H.

6. Complex according to any of Claims 1 to 5,
wherein the compound of formula (I) has the structure (1) :

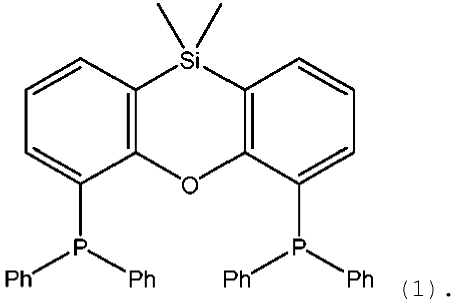

7. Complex according to any of Claims 1 to 6,
wherein the complex has exactly one ligand corresponding to formula (I).

8. Complex according to any of Claims 1 to 7,
wherein the complex has at least two iodine ligands.

9. Complex according to Claim 8,
wherein the complex has exactly two iodine ligands.

10. Complex according to any of Claims 1 to 7,
wherein the complex has at least two bromine ligands.

11. Complex according to Claim 10,
wherein the complex has exactly two bromine ligands.

12. Use of a complex according to any of Claims 1 to 11 for catalysis of a hydroformylation reaction.

## Revendications

1. Complexe comprenant :
a) du Pt;
b) un ligand selon la formule (I) : 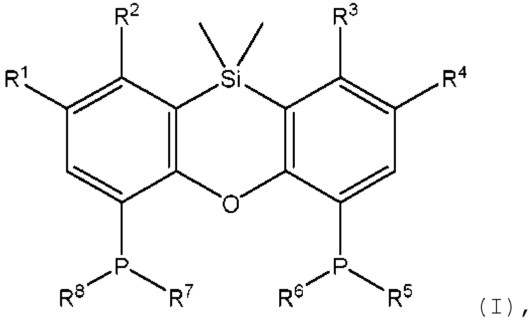 dans laquelle R¹, R², R³, R⁴, R⁵, R⁶, R⁷, R⁸ sont choisis parmi : -H, - (C₁-C₁₂)-alkyle, -(C₆-C₂₀)-aryle ;
c) un ligand iode ou un ligand brome.

2. Complexe selon la revendication 1,
R⁵, R⁶, R⁷, R⁸ représentant -(C₆-C₂₀)-aryle.

3. Complexe selon l'une des revendications 1 ou 2,
R⁵, R⁶, R⁷, R⁸ représentant -Ph.

4. Complexe selon l'une des revendications 1 à 3,
R¹ et R⁴ représentant -H.

5. Complexe selon l'une des revendications 1 à 4,
R² et R³ représentant -H.

6. Complexe selon l'une des revendications 1 à 5,
le composé selon la formule (I) présentant la structure (1) :

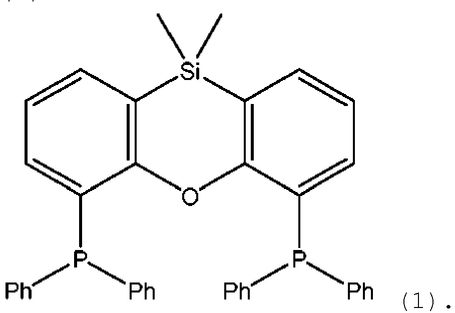

7. Complexe selon l'une des revendications 1 à 6,
le complexe présentant exactement un ligand selon la formule (I).

8. Complexe selon l'une des revendications 1 à 7,
le complexe présentant au moins deux ligands iode.

9. Complexe selon la revendication 8,
le complexe présentant exactement deux ligands iode.

10. Complexe selon l'une des revendications 1 à 7,
le complexe présentant au moins deux ligands brome.

11. Complexe selon la revendication 10,
le complexe présentant exactement deux ligands brome.

12. Utilisation d'un complexe selon l'une des revendications 1 à 11 pour la catalyse d'une réaction d'hydroformylation.
